# EUROPEAN PATENT SPECIFICATION

(11) **EP 3 079 570 B1**
(45) Date of publication and mention of the grant of the patent: **28.03.2018**
(21) Application number: 14814694.7
(22) Date of filing: 11.12.2014
(51) Int. Cl.: A61B 5/02, A61B 5/021

(54) **SYSTEM AND METHOD FOR MEASURING A PULSE WAVE OF A SUBJECT**
VORRICHTUNG UND VERFAHREN ZUR MESSUNG DER PULSWELLE EINER PERSON
APPAREIL ET PROCÉDÉ POUR MESURER UNE ONDE D'IMPULSION D'UN SUJET

(30) Priority: 11.12.2013 WO PCT/CN2013/089040; 27.02.2014 EP 14156897
(43) Date of publication of application: 19.10.2016
(73) Proprietor: Koninklijke Philips N.V., 5656 AE Eindhoven (NL)
(72) Inventor: VIELLARD-BOUTRY, Clementine Marie Françoise, NL-5656 AE Eindhoven (NL); MOSIS, Georgio, NL-5656 AE Eindhoven (NL); BERA, Deep, NL-5656 AE Eindhoven (NL); MO, Weirong, NL-5656 AE Eindhoven (NL); QIAN, Yi, NL-5656 AE Eindhoven (NL)
(74) Representative: Gravendeel, Cornelis
(86) International application number: PCT/EP2014/077327
(87) International publication number: WO 2015/086725

(56) References cited:
- US-A1- 2004 039 420
- SUGAWARA J ET AL: "Carotid-femoral pulse wave velocity: Impact of different arterial path length measurements", ARTERY RESEARCH, ELSEVIER, AMSTERDAM, NL, vol. 4, no. 1, 1 March 2010 (2010-03-01), pages 27-31, XP026938470, ISSN: 1872-9312, DOI: 10.1016/J.ARTRES.2009.11.001 [retrieved on 2009-12-04]
- CORTEZ-COOPER M Y ET AL: "Effects of High Intensity Resistance Training on Arterial Stiffness and Wave Reflection in Women", AMERICAN JOURNAL OF HYPERTENSION, NATURE PUBLISHING GROUP, UNITED STATES, vol. 18, no. 7, 1 July 2005 (2005-07-01), pages 930-934, XP027816786, ISSN: 0895-7061 [retrieved on 2005-07-01]

## Description

### FIELD OF THE INVENTION

The present invention relates to biological measurement, in particular to a system and method for measuring a pulse wave of a subject.

### BACKGROUND OF THE INVENTION

Pulse wave measurements play a more and more important role in advanced medical detection. As an example of pulse wave measurements, pulse wave velocity (PWV) measurements are used to assess arterial stiffness, which is one of the standards for evaluating hypertension and cardiovascular diseases (CVD). More specifically, carotid-femoral pulse wave velocity (cfPWV) is regarded as a "golden standard" to evaluate aortic arteriosclerosis.

PWV is defined as a distance between two artery measurement points divided by a pulse transit time during which a pulse wave of a subject propagates between the two artery measurement points. For example, cfPWV is defined as a distance between a measurement point on skin above a carotid of a subject and a measurement point on skin above a femoral of the subject divided by a pulse transit time during which a pulse of the subject propagates between the two artery measurement points above respectively the carotid and the femoral.

Therefore, in order to measure the cfPWV of the subject, first the two artery measurement points on skin of the subject are located above respectively the carotid and the femoral. Then, the distance between the two artery measurement points above respectively the carotid and the femoral is measured by e.g. a ruler. In order to calculate the pulse transit time during which a pulse of the subject propagates between the two artery measurement points above respectively the carotid and the femoral, a periodic cycle detection algorithm is first applied to the two pulse wave signals detected at the two artery measurement points to detect the periods of the two pulse wave signals respectively, and then the pulse transit time is derived based on the periods of the two pulse wave signals. Then, cfPWV can be derived from the distance between the two artery measurement points and the derived pulse transit time.

In the prior art, in e.g. cfPWV measurement, a doctor or a nurse finds the measurement points above the carotid and the femoral by touch. This is time consuming and the result may not be accurate.

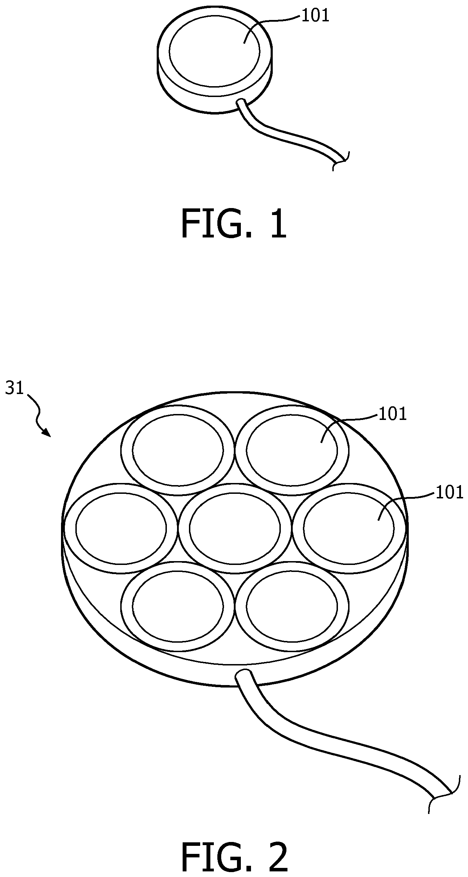

In the prior art, in e.g. cfPWV measurement, for each of the carotid and the femoral, a sensor 101 (usually a piezo-electric sensor) as shown in Fig. 1 is used to sense the pulse at the corresponding measurement point to derive the corresponding pulse wave signal. However, because the measurement point where the sensor 101 is to be placed is found by touch by the doctor or the nurse, the corresponding pulse wave signal may not represent the strongest pulse for e.g. the carotid or the femoral, thus rendering the measurement result (e.g. the pulse transit time calculation result) inaccurate.

"Carotid-femoral pulse wave velocity: Impact of different arterial path length measurements"(SUGAWARA J ET AL, ARTERY RESEARCH, ELSEVIER, AMSTERDAM, NL, vol. 4, no. 1, 1 March 2010 (2010-03-01), pages 27-31, XP026938470,ISSN: 1872-9312, 001: 10.1016/J.ARTRES.2009.11.001) discloses a method for Carotid-femoral PWV calculation: (1) the straight distance between carotid and femoral sites (PWVcar-fem), (2) the straight distance between suprasternal notch and femoral site minus carotid arterial length (PWV(ssn^fem)-(ssn-car)), (3) the straight distance between carotid and femoral sites minus carotid arterial length (PWV(car-fem)∼(ssn-car)), and (4) the combined distance from suprasternal notch to the umbilicus and from the umbilicus to femoral site minus carotid arterial length (PWV(ssn_amb-fem)-(ssn-car)).

"Assessment of pulse wave velocity"(BOUTOUYRIE P ET AL, ARTERY RESEARCH, ELSEVIER, AMSTERDAM, NL, vol. 3, no. 1, 1 February 2009 (2009-02-01), pages 3-8, XP025972566,ISSN: 1872-9312, DOI: 10.1016/J.ARTRES.2008.11.002) gives a brief overlook of the marketed devices to measure pulse wave velocity.

### SUMMARY OF THE INVENTION

Therefore, it would be advantageous to provide a system and method for measuring a pulse wave of a subject, which can improve efficiency and/or accuracy of pulse wave measurement.

The invention is defined in independent claims 1 and 11 and the dependent claims 2 - 10.

According to an embodiment of this invention, a system is proposed for measuring a pulse wave of a subject, comprising: a first sensor array and a second sensor array for respectively sensing a corresponding pulse wave of a first and a second artery of the subject when being placed on the skin of the subject above respectively the first artery and the second artery of the subject, each of the first sensor array and the second sensor array comprising a plurality of sensors for respectively acquiring a plurality of first signals indicating a vibration of the skin; a deriving unit configured to derive, for each of the first sensor array and the second sensor array, a second signal representing the corresponding pulse wave from the plurality of first signals; and a first calculating unit configured to calculate a pulse transit time between the first artery and the second artery from the second signal derived for the first sensor array and the second signal derived for the second sensor array.

Accordingly, a sensor array is used for each of the pulse wave measurements of the first artery and the second artery (e.g. the carotid and the femoral). Each sensor array comprises a plurality of sensors rather than a single sensor. Since the sensor array can cover a larger skin area than a single sensor, the pulse wave can be sensed in a qualitatively good way without the need for very accurate sensor positioning as is required when using a single sensor. Thus, the positioning of the sensor array can be done more efficiently, and the quality of the measurement is less dependent on the skills of the doctor or the nurse, as compared to the situation in the prior art where only a single sensor is used.

In an embodiment, each of the plurality of sensors comprises a piezo-electric sensor.

In an embodiment, each of the plurality of sensors comprises an accelerometer.

The advantages of using an accelerometer instead of a piezo-electric sensor according to an embodiment of this invention are the following:
First, an accelerometer is more flexible to adapt to local topography of the skin as compared to a piezoelectric sensor. Each of the sensors in the sensor array has a contact surface to contact skin of the subject through an appropriate mechanical coupling. It means that all the contact surfaces of the sensors have to be perfectly aligned to each other, and that the overall measurement sensor array should be flexible enough so that it adapts to the local topography of the skin. However, because of the design of the piezoelectric sensors (each sensor consisting of a membrane fixed on a rigid cylindrical packaging), it is very rigid. Accelerometers, by contrast, could be fixed on a flexible substrate, thus solving this rigidity issue.

Second, it is easier to integrate a lot of accelerometers into one sensor array than a lot of piezoelectric sensors. It would be of interest to integrate a larger number of sensors into the sensor array, in order to be able to accurately locate the signal which represents the strongest pulse for e.g. carotid or femoral. While such an integration poses quite a challenge with piezoelectric sensors, it is straightforward with accelerometers.

Third, the measurement result provided by accelerometers is not influenced by the way the operator holds the sensor array and thus is more reliable than that provided by piezoelectric sensors, which tends to be influenced by the way the operator holds the sensor array.

Fourth, a frequency range of interest for PWV measurement (DC through 40-50Hz) can be covered by an efficient operating frequency of accelerometers, but not by an efficient operating frequency of the piezoelectric sensors (0.2Hz through 40-50Hz).

Preferably, the accelerometer is a micro-machined resistive or capacitive accelerometer. The micro-machined resistive accelerometer can be a strain gauge, piezo resistive, MEMS (Micro Electro Mechanical Systems), or thin-film accelerometer.

Using a micro-machined resistive or capacitive accelerometer can offer several advantages. For example, it is small and lightweight, which allows manufacturing an array of accelerometers on a flexible membrane. The BOM (bill of material) cost is low. It is very suitable for pulse wave measurement, which requires a measurement from DC to about 50 Hz, because it can measure down to 0 Hz and its limited high frequency range of about 10 kHz has no negative impact on pulse wave measurement. It can provide high sensitivity. It is robust and typically used for long duration events, thereby being able to withstand reasonable levels of shock in the real world environment of the hospital.

In an embodiment, each of the plurality of sensors is configured to acquire the first signals indicating vibration of the skin in at least two directions. For example, a 3D-axis accelerometer can acquire a signal in three directions perpendicular to each other in a rectangular coordinate system.

As compared to a one-direction signal sensed by a one-direction sensor, the additional information contained in a multi-direction signal obtained by a multi-direction sensor can be used to eliminate artifacts associated e.g. with an operator's involuntary movements, thus improving measurement accuracy.

In an embodiment, each of the plurality of sensors comprises a contact surface for contacting the skin of the subject; the at least two directions comprise an X-axis direction which is perpendicular to the contact surface, and the first deriving unit is configured, for each of the first sensor array and the second sensor array, to select a first signal from the plurality of first signals, and to derive the second signal from the X-axis direction component of the selected first signal.

In an embodiment, the at least two directions further comprise a Z-axis direction perpendicular to the X-axis direction. The deriving unit is configured, for each of the first sensor array and the second sensor array, to derive the second signal by subtracting the Z-axis direction component of the selected first signal from the X-axis direction component of the selected first signal.

In this way, the pulse wave signal can be derived and the noise therein can be reduced. This is based on the following idea. The X-axis is the direction perpendicular to the contact surface and is substantially perpendicular to the skin surface when the sensor array is placed on the skin, and therefore the X-axis can be viewed as the vibration direction of the pulse wave, and the X-axis component of the signal is expected to represent the pulse wave plus noise in the pulse direction. Similarly, the Z-axis is substantially perpendicular to the vibration direction of the pulse wave, and therefore, the Z-axis component of the signal is expected to mainly reflect noise and contain no pulse information. Under the assumption that the noise is uniformly distributed in all directions, the signal representing the pulse wave can be derived by subtracting the Z-axis component from the X-axis component of the signal.

In an embodiment, selecting a first signal from the plurality of first signals may be implemented in the following way: the deriving unit is configured to determine, among the plurality of first signals, a group of first signals, wherein all cross-correlations of the X-axis direction component of each of the first signals in the group with the X-axis direction components of other first signals in the group are above a first predetermined threshold, and all cross-correlations of the X-axis direction component of each of the first signals in the group with the X-axis direction components of the first signals outside the group are below a second predetermined threshold, and select the first signal from the first signals in the group at least based on the signal amplitude of the first signals. For example, the signal amplitude or signal to noise ratio of the selected first signal is the highest among the first signals in the group.

Among the first signals acquired by the plurality of sensors, there is a first type of first signals whose X-axis direction components mainly contain a sensed pulse and may contain a little noise, which signals are useful for deriving the signal representing the strongest pulse (i.e. the second signal), and there is also a second type of first signals whose X-axis direction components mainly consist of noise and which have a low degree of useful information reflecting the pulse and which should be removed when deriving the signal representing the strongest pulse (i.e. the second signal). Because the pulse is not correlated with the noises, and nearly all the noises are not correlated among each other due to the nature of the pulse and the noise, the X-axis direction components of any two signals among the first type of first signals mainly consisting of a sensed pulse have a high cross-correlation, the X-axis direction component of any signal among the first type of first signals mainly consisting of a sensed pulse and the X-axis direction component of any signal among the second type of first signals mainly consisting of noise have a low cross-correlation, and X-axis direction components of any two signals among the second type of first signals mainly consisting of noise have a low cross-correlation. Using the advantages thereof, it is proposed to first determine a group of first signals, wherein all cross-correlations of the X-axis direction component of each of the first signals in the group with the X-axis direction components of other first signals in the group are above a first predetermined threshold, and all cross-correlations of the X-axis direction component of each of the first signals in the group with the X-axis direction components of the first signals outside the group are below a second predetermined threshold. The first predetermined threshold and the second predetermined threshold are selected according to practical requirements, which can be determined in practice. In this way, each of the first signals in the determined group is expected to have a pulse content which is larger than the noise content.

Furthermore, selecting a first signal based on at least the signal amplitude of the first signal can improve accuracy of pulse wave measurement. In an example, the signal amplitude of the selected first signal is the highest among the first signals in the group. In another example, the signal to noise ratio of the selected first signal is the highest among the first signals in the group.

In another embodiment, the deriving unit is configured to derive, for each of the plurality of first signals, a component of the first signal representing a vibration substantially along the vibration of the pulse wave.

In an embodiment, each sensor array comprises a packaging case with a membrane for contacting the skin of the subject. The plurality of sensors are fixed on one of the two sides of the membrane enclosed within the packaging case, and the other side of the membrane is to be placed on the skin of the subject.

Because, in this embodiment, the plurality of sensors are not in direct contact with the skin of a subject and are encapsulated within the packaging case, the cleaning procedure is simplified. Moreover, because the plurality of sensors do not contact the skin of a subject directly, it makes it possible for the sensors to acquire the plurality of first signals in at least two directions, e.g. in 3D detection. Furthermore, such fixation of the sensors on the membrane guarantees optimal coupling between the skin and the membrane.

Preferably, the membrane is a thin metal membrane.

If the membrane is not a viscoelastic membrane, it will add noise to the recorded sensor signal. Therefore, according to an embodiment of the present invention, a thin metal membrane which is viscoelastic is used to reduce noise. Thus, the advantageous effect of noise reduction in the sensed signal sensed by the sensors can be achieved.

In an embodiment, the system further comprises: an obtaining unit configured to obtain a parameter indicating a distance travelled by the pulse wave between the first artery and the second artery; and a second calculating unit configured to calculate a pulse wave velocity of the subject based on the calculated pulse transit time and the obtained parameter.

According to another aspect of this invention, a method is proposed for measuring a pulse wave of a subject comprising: placing a first sensor array and a second sensor array respectively on the skin of the subject above a first artery and a second artery of the subject for respectively sensing a corresponding pulse wave of the first and the second artery of the subject, each of the first sensor array and the second sensor array comprising a plurality of sensors for respectively acquiring a plurality of first signals indicating a vibration of the skin; deriving, for each of the first sensor array and the second sensor array, a second signal representing the corresponding pulse wave from the plurality of first signals; and calculating a pulse transit time between the first artery and the second artery from the second signal derived for the first sensor array and the second signal derived for the second sensor array.

Various aspects and features of the disclosure are described in further detail below. These and other aspects of the invention will be apparent from and elucidated with reference to the embodiment(s) described hereinafter.

### DESCRIPTION OF THE DRAWINGS

The present invention will be described and explained hereinafter in more detail in combination with embodiments and with reference to the drawings, wherein:
Fig. 1 shows a prior-art sensor for sensing the pulse at the corresponding measurement point to derive the corresponding pulse wave signal.
Fig. 2 shows a sensor array comprising a plurality of sensors according to an embodiment of this invention.
Fig. 3 shows a block diagram of a system for measuring a pulse wave of a subject according to an embodiment of this invention.
Figs. 4a-b show two types of structure of the first sensor array or a second sensor array according to embodiments of this invention.
Fig. 5 shows a block diagram of a method for measuring a pulse wave of a subject according to an embodiment of this invention.

The same reference signs in the figures indicate similar or corresponding features and/or functionalities.

### DETAILED DESCRIPTION OF THE EMBODIMENTS

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn to scale for illustrative purposes.

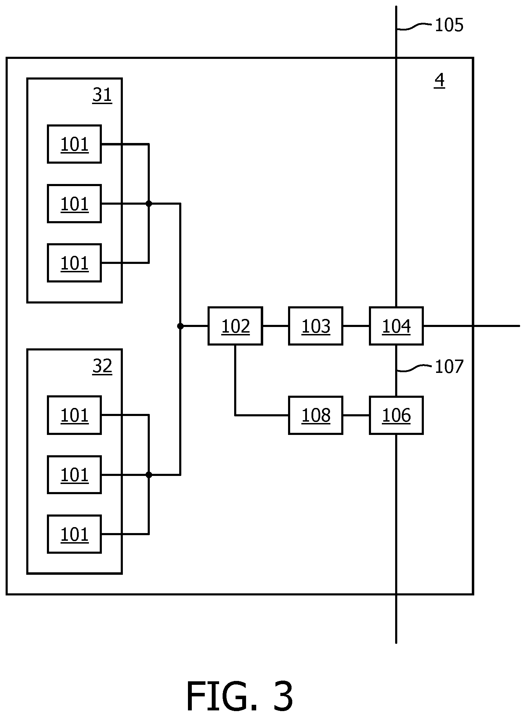

Fig. 3 shows a block diagram of a system 4 for measuring a pulse wave of a subject according to an embodiment of this invention. The system 4 comprises a first sensor array 31, a second sensor array 32, a deriving unit 102, a first calculating unit 103 and, optionally, a second calculating unit 104, an obtaining unit 106 and a determining unit 108.

The first sensor array 31 and the second sensor array 32 are to be respectively placed on the skin of the subject above a first artery (e.g. carotid) and a second artery (e.g. femoral) for sensing a corresponding pulse wave of, respectively, the first and the second artery of the subject. By the corresponding pulse wave of an artery is meant a pulse wave at a measurement point on the skin above the artery. Therefore, the corresponding pulse wave of the first artery means a pulse wave at a first measurement point on the skin above the first artery, and the corresponding pulse wave of the second artery means a pulse wave at a second measurement point on the skin above the second artery.

As illustrated in Fig. 2, the first sensor array 31 comprises a plurality of sensors 101 for respectively acquiring a plurality of first signals indicating a vibration of the skin. Similarly, the second sensor array 32 also comprises a plurality of sensors 101 for respectively acquiring a plurality of first signals indicating a vibration of the skin.

The deriving unit 102 derives, for each of the first sensor array 31 and the second sensor array 32, a second signal representing the corresponding pulse wave from the plurality of first signals. The signal-deriving procedure will be discussed in greater detail later in this description.

The first calculating unit 103 calculates a pulse transit time between the first artery and the second artery from the second signal derived for the first sensor array 31 and the second signal derived for the second sensor array 32. For example, as stated in the prior art, a periodic cycle detection algorithm may be first applied to the second signal derived for the first sensor array 31 and the second signal derived for the second sensor array 32 to detect the periods of the second signal derived for the first sensor array 31 and the periods of the second signal derived for the second sensor array 32, and then the pulse transit time is derived based on the two detected periods.

The obtaining unit 106 obtains a parameter 107 indicating a distance travelled by the pulse wave between the first artery and the second artery. It can be embodied in many ways. For example, it can be only a receipt unit, which receives the parameter 107 input by an operator. As another example, it can be an electronic ruler which automatically measures the distance travelled by the pulse wave between the first artery and the second artery. Of course, in order to enhance pulse-wave-velocity-calculation accuracy, the second signal derived by the deriving unit 102 for the first sensor array 31 and the second signal derived by the deriving unit 102 for the second sensor array 32 are input to the determining unit 108, which determines the measurement point corresponding to the second signal derived for the first sensor array 31 and the measurement point corresponding to the second signal derived for the first sensor array 32 and outputs the two determined measurement points to the electronic ruler, which then measures the distance on the skin of the subject between the two determined measurement points and outputs the parameter 107 indicating a distance travelled by the pulse wave between the first artery and the second artery to the second calculating unit 104.

The second calculating unit 104 calculates a pulse wave velocity 105 of the subject based on the calculated pulse transit time and the obtained parameter 107. The pulse wave velocity 105 is equal to the obtained parameter 107 indicating a distance travelled by the pulse wave between the first artery and the second artery divided by the calculated pulse transit time.

Each of the plurality of sensors 101 may be a piezo-electric sensor or an accelerometer, preferably, a micro-machined resistive or capacitive accelerometer.

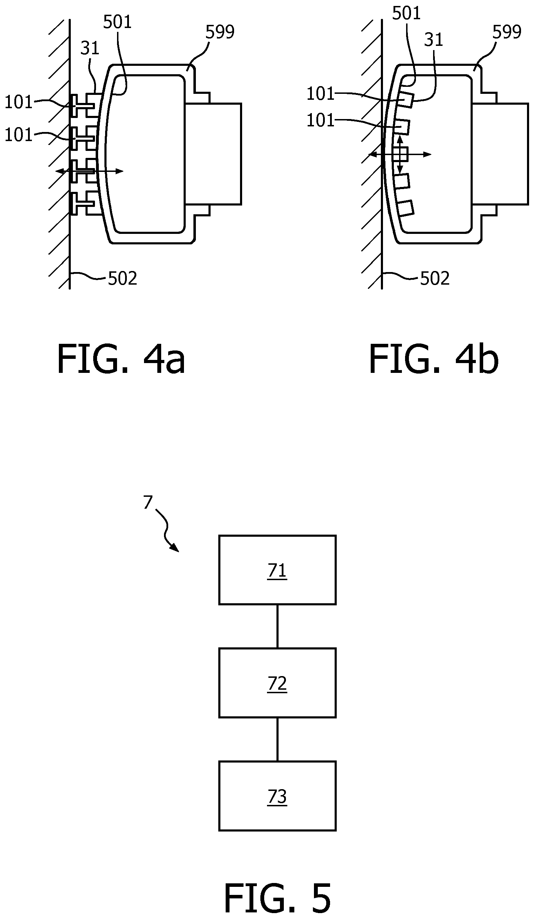

Fig. 4a shows a type of structure of the first sensor array 31 or a second sensor array 32 according to an embodiment of this invention.

In Fig. 4a, the sensor array comprises a packaging case 599 with a flexible membrane 501 on which the plurality of sensors 101 are fixed. The flexible membrane does not contact the skin of the subject directly. For each sensor, a piston is connected to the moving mass of the sensor, and is placed in mechanical contact with the skin of the subject, above the first artery or the second artery. The mechanical vibrations coming from the pulse of the first artery or the second artery are transmitted through each piston to a sensing body of the corresponding sensor. In Fig. 4a, each of the sensors 101 only acquires a first signal in one direction, i.e. a direction perpendicular to the skin.

The plurality of first signals acquired by the plurality of sensors 101 are input to the deriving unit 102. Among the plurality of first signals, the deriving unit 102 determines a group of first signals, wherein all cross-correlations of each of the first signals in the group with other first signals in the group are above a first predetermined threshold, and all cross-correlations of each of the first signals in the group with the first signals outside the group are below a second predetermined threshold.

Then, a first signal is selected as the second signal representing the corresponding pulse wave among this group of first signals, at least based on amplitudes. For example, the first signal with the highest amplitude or SNR, etc. is selected.

The advantage of the embodiment of Fig. 4a resides in direct contact between the sensor and the skin, thus optimizing coupling and pulse transmission efficiency.

Fig. 4b shows another type of structure of the first sensor array 31 or a second sensor array 32 according to an embodiment of this invention.

In Fig. 4b, each sensor array comprises a packaging case 599 with a membrane 501 for contacting the skin 502 of the subject. The plurality of sensors 101 are fixed on one of the two sides of a membrane 501 (preferably a thin metal membrane, as discussed above) enclosed within the packaging case 599, and the other side of the membrane 501 which is not enclosed within the packaging case 599 is to be placed on the skin 502 of the subject.

In one embodiment, each of the plurality of sensors has a contact surface for contacting the skin of the subject. The contact may be direct or indirect contact. As shown in Fig. 4b, the contact takes place via the membrane 501. The at least two directions comprise an X-axis direction, which is a direction perpendicular to the contact surface. The at least two directions may further comprise a Y-axis direction and a Z-axis direction, which are defined as directions perpendicular to the X-axis direction and perpendicular to each other in order to constitute a rectangular coordinate system together with the X-axis. For example, the Y-axis direction is defined as the direction parallel to the contact surface, and the Z-axis direction is defined as perpendicular to the X-axis direction and the Y-axis direction.

The deriving unit 102 is configured, for each of the first sensor array 31 and the second sensor array 32, to select a first signal from the plurality of first signals, and to derive the second signal from the X-axis direction component of the selected first signal. The selecting procedure is e.g. as follows: among the plurality of first signals, the deriving unit 102 determines a group of first signals, wherein all cross-correlations of the X-axis direction component of each of the first signals in the group with the X-axis direction components of other first signals in the group are above a first predetermined threshold, and all cross-correlations of the X-axis direction component of each of the first signals in the group with the X-axis direction components of the first signals outside the group are below a second predetermined threshold, and selects the first signal from the first signals in the group at least based on the signal amplitude of the first signal. For example, the signal amplitude or signal to noise ratio of the selected first signal is the highest among the first signals in the group.

Then, for each of the first sensor array 31 and the second sensor array 32, the deriving unit 102 derives the second signal by subtracting the Z-axis direction component of the selected first signal from the X-axis direction component of the selected first signal.

In another embodiment, the deriving unit 102 derives, for each of the plurality of first signals, a component of the first signal representing a vibration substantially along the vibration of the pulse wave. This can be done by a known eigenvector decomposition or principle component analysis method.

Fig. 5 shows a block diagram of a method 7 for measuring a pulse wave of a subject according to an embodiment of this invention. The method 7 comprises: at step 71, placing a first sensor array 31 and a second sensor array 32 respectively on the skin of the subject above a first artery and a second artery of the subject for respectively sensing a corresponding pulse wave of the first and the second artery of the subject, each of the first sensor array and the second sensor array comprising a plurality of sensors 101 for respectively acquiring a plurality of first signals indicating a vibration of the skin; at step 72, deriving, for each of the first sensor array 31 and the second sensor array 32, a second signal representing the corresponding pulse wave from the plurality of first signals; and at step 73, calculating a pulse transit time between the first artery and the second artery from the second signal derived for the first sensor array 31 and the second signal derived for the second sensor array 32. It is to be noted that the steps of the methods shown in the present invention should not be limited to the steps mentioned above. It will be apparent to those skilled in the art that the various aspects of the invention claimed may be practiced in other examples that depart from these specific details.

Furthermore, as can be easily understood by the person skilled in the art, the deriving unit 102, the first calculating unit 103 and the second calculating unit 104, etc. in Fig. 3 can be embodied by one and the same item of hardware. Of course, they can be realized as separate items of hardware. Further, the deriving unit 102, the first calculating unit 103 and the second calculating unit 104, etc. in Fig. 3 can be realized in software instructions, which can be loaded to a general computer having e.g. a CPU, a memory, a display and an I/O interface, so that the CPU in the general computer could perform the instructions to carry out any functions of one or more of the deriving unit 102, the first calculating unit 103 and the second calculating unit 104, etc. in Fig. 3.

The mere fact that certain measures are recited in mutually different dependent claims does not indicate that a combination of these measures cannot be used to advantage.

It should be noted that the above-mentioned embodiments illustrate rather than limit the invention and that those skilled in the art will be able to design alternative embodiments without departing from the scope of the appended claims. In the claims, any reference signs placed between parentheses shall not be construed as limiting the claim. The word "comprising" does not exclude the presence of elements or steps not listed in a claim or in the description. The word "a" or "an" preceding an element does not exclude the presence of a plurality of such elements. In the system claims enumerating several units, several of these units can be embodied by one and the same item of software and/or hardware. The usage of the words first, second and third, et cetera, does not indicate any ordering. These words are to be interpreted as names.

## Claims

1. A system (4) for measuring a pulse wave of a subject, comprising:
a first sensor array (31) and a second sensor array (32) for respectively sensing a corresponding pulse wave of a first and a second artery of the subject when being placed on the skin of the subject above respectively the first artery and the second artery of the subject, each of the first sensor array and the second sensor array comprising a plurality of sensors (101) for respectively acquiring a plurality of first signals indicating a vibration of the skin;
a deriving unit (102) configured to derive, for each of the first sensor array (31) and the second sensor array(32), a second signal representing the corresponding pulse wave from the plurality of first signals; and
a first calculating unit (103) configured to calculate a pulse transit time between the first artery and the second artery from the second signal derived for the first sensor array (31) and the second signal derived for the second sensor array (32); **characterized in that**:
each of the plurality of sensors (101) is configured to acquire the first signal indicating the vibration of the skin in at least two directions;
wherein
each of the plurality of sensors (101) comprises a contact surface for contacting the skin of the subject;
the at least two directions comprise an X-axis direction, which is a direction perpendicular to the contact surface; and
the deriving unit (102) is configured, for each of the first sensor array (31) and the second sensor array (32), to select a first signal from the plurality of first signals, and to derive the second signal from the X-axis direction component of the selected first signal.

2. The system (4) according to claim 1, wherein each of the plurality of sensors (101) comprises an accelerometer.

3. The system (4) according to claim 2, wherein the accelerometer is a micro-machined resistive or capacitive accelerometer.

4. The system (4) according to claim 1, wherein
the at least two directions further comprise a Z-axis direction perpendicular to the X-axis direction; and
the deriving unit (102) is configured, for each of the first sensor array (31) and the second sensor array (32), to derive the second signal by subtracting the Z-axis direction component of the selected first signal from the X-axis direction component of the selected first signal.

5. The system (4) according to claim 1, wherein for each of the first sensor array (31) and the second sensor array (32), the deriving unit (102) is configured to:
determine, among the plurality of first signals, a group of first signals, wherein all cross-correlations of the X-axis direction component of each of the first signals in the group with the X-axis direction components of other first signals in the group are above a first predetermined threshold, and all cross-correlations of the X-axis direction component of each of the first signals in the group with the X-axis direction components of the first signals outside the group are below a second predetermined threshold , and
select the first signal from the first signals in the group at least based on the signal amplitude of the first signals.

6. The system (4) according to claim 5, wherein the signal amplitude or signal to noise ratio of the selected first signal is the highest among the first signals in the group.

7. The system (4) according to claim 1, wherein the deriving unit (102) is configured to derive, for each of the plurality of first signals, a component of the first signal representing a vibration substantially along the vibration of the pulse wave.

8. The system (4) according to claim 1, wherein the plurality of sensors (101) are fixed on one of the two sides of a membrane and the other side of the membrane is to be placed on the skin of the subject.

9. The system (4) according to claim 8, wherein the membrane is a thin metal membrane.

10. The system (4) according to claim 1, further comprising:
an obtaining unit (106) configured to obtain a parameter (107) indicating a distance travelled by the pulse wave between the first artery and the second artery; and
a second calculating unit (104) configured to calculate a pulse wave velocity (105) of the subject, based on the calculated pulse transit time and the obtained parameter.

11. A method (7) of measuring a pulse wave of a subject, comprising:
placing (71) a first sensor array (31) and a second sensor array (32) respectively on the skin of the subject above a first artery and a second artery of the subject for respectively sensing a corresponding pulse wave of the first and the second artery of the subject, each of the first sensor array and the second sensor array comprising a plurality of sensors (101) for respectively acquiring a plurality of first signals indicating a vibration of the skin;
deriving (72), for each of the first sensor array (31) and the second sensor array (32), a second signal representing the corresponding pulse wave from the plurality of first signals; and
calculating (73) a pulse transit time between the first artery and the second artery from the second signal derived for the first sensor array (31) and the second signal derived for the second sensor array (32); **characterized in that**:
each of the plurality of sensors (101) is configured to acquire the first signal indicating the vibration of the skin in at least two directions;
wherein
each of the plurality of sensors (101) comprises a contact surface for contacting the skin of the subject;
the at least two directions comprise an X-axis direction, which is a direction perpendicular to the contact surface; and
the deriving unit (102) is configured, for each of the first sensor array (31) and the second sensor array (32), to select a first signal from the plurality of first signals, and to derive the second signal from the X-axis direction component of the selected first signal.

## Patentansprüche

1. System (4) zur Messung einer Pulswelle einer Person, umfassend:
ein erstes Sensorarray (31) und ein zweites Sensorarray (32) zum Abtasten einer zugehörigen Pulswelle einer ersten bzw. einer zweiten Arterie der Person, wenn sie auf der Haut der Person über der ersten Arterie bzw. der zweiten Arterie der Person platziert werden, wobei sowohl das erste Sensorarray als auch das zweite Sensorarray eine Vielzahl von Sensoren (101) umfassen, um jeweils eine Vielzahl von ersten Signalen zu erfassen, die eine Vibration der Haut angeben;
eine Ableitungseinheit (102), die konfiguriert ist, um sowohl für das erste Sensorarray (31) als auch für das zweite Sensorarray (32) ein zweites Signal, das die zugehörige Pulswelle darstellt, von der Vielzahl von ersten Signalen abzuleiten; und
eine erste Berechnungseinheit (103), die konfiguriert ist, um eine Pulswellenlaufzeit zwischen der ersten Arterie und der zweiten Arterie anhand des für das erste Sensorarray (31) abgeleiteten zweiten Signals und des für das zweite Sensorarray (32) abgeleiteten zweiten Signals zu berechnen;
**dadurch gekennzeichnet, dass**:
jeder der Vielzahl von Sensoren (101) konfiguriert ist, um das erste Signal, das die Vibration der Haut angibt, in mindestens zwei Richtungen zu erfassen;
wobei
jeder der Vielzahl von Sensoren (101) eine Kontaktfläche zum Kontaktieren der Haut der Person umfasst;
die mindestens zwei Richtungen eine X-Achsen-Richtung umfassen, die eine Richtung senkrecht zu der Kontaktfläche ist; und
die Ableitungseinheit (102) konfiguriert ist, um für sowohl das erste Sensorarray (31) als auch das zweite Sensorarray (32) ein erstes Signal aus der Vielzahl von ersten Signalen auszuwählen, und um das zweite Signal von der X-Achsen-Richtungskomponente des ausgewählten ersten Signals abzuleiten.

2. System (4) nach Anspruch 1, wobei jeder der Vielzahl von Sensoren (101) einen Beschleunigungsmesser umfasst.

3. System (4) nach Anspruch 2, wobei der Beschleunigungsmesser ein mikrobearbeiteter resistiver oder kapazitiver Beschleunigungsmesser ist.

4. System (4) nach Anspruch 1, wobei
die mindestens zwei Richtungen weiterhin eine Z-Achsen-Richtung senkrecht zur X-Achsen-Richtung umfassen; und
die Ableitungseinheit (102) konfiguriert ist, um für sowohl das erste Sensorarray (31) als auch das zweite Sensorarray (32) das zweite Sensorsignal durch Subtrahieren der Z-Achsen-Richtungskomponente des ausgewählten ersten Signals von der X-Achsen-Richtungskomponente des ausgewählten ersten Signals abzuleiten.

5. System (4) nach Anspruch 1, wobei die Ableitungseinheit (102) für sowohl das erste Sensorarray (31) als auch das zweite Sensorarray (32) konfiguriert ist, um:
aus der Vielzahl von ersten Signalen eine Gruppe von ersten Signalen zu ermitteln, wobei alle Kreuzkorrelationen der X-Achsen-Richtungskomponente von jedem der ersten Signale in der Gruppe mit den X-Achsen-Richtungskomponenten der anderen ersten Signale in der Gruppe über einem ersten vorgegebenen Schwellenwert liegen, und alle Kreuzkorrelationen der X-Achsen-Richtungskomponente von jedem der ersten Signale in der Gruppe mit den X-Achsen-Richtungskomponenten der ersten Signale außerhalb der Gruppe unter einem zweiten vorgegebenen Schwellenwert liegen, und
das erste Signal aus den ersten Signalen in der Gruppe zumindest basierend auf der Signalamplitude der ersten Signale auszuwählen.

6. System (4) nach Anspruch 5, wobei die Signalamplitude oder der Störabstand des ausgewählten ersten Signals unter den ersten Signalen in der Gruppe am höchsten ist.

7. System (4) nach Anspruch 1, wobei die Ableitungseinheit (102) konfiguriert ist, um für jedes der Vielzahl von ersten Signalen eine Komponente des ersten Signals, das eine Vibration darstellt, im Wesentlichen entlang der Vibration der Pulswelle abzuleiten.

8. System (4) nach Anspruch 1, wobei die Vielzahl von Sensoren (101) auf einer der beiden Seiten einer Membran befestigt sind und die andere Seite der Membran auf der Haut der Person zu platzieren ist.

9. System (4) nach Anspruch 8, wobei die Membran eine dünne Metallmembran ist.

10. System (4) nach Anspruch 1, weiterhin umfassend:
eine Erlangungseinheit (106), die konfiguriert ist, um einen Parameter (107) zu erlangen, der eine durch die Pulswelle zwischen der ersten Arterie und der zweiten Arterie zurückgelegte Distanz angibt; und
eine zweite Berechnungseinheit (104), die konfiguriert ist, um eine Pulswellengeschwindigkeit (105) der Person basierend auf der Pulswellenlaufzeit und dem erlangten Parameter zu berechnen.

11. Verfahren (7) zur Messung einer Pulswelle einer Person, umfassend:
Platzieren (71) eines ersten Sensorarrays (31) und eines zweiten Sensorarrays (32) auf der Haut der Person über einer ersten Arterie bzw. einer zweiten Arterie der Person zum Abtasten einer zugehörigen Pulswelle der ersten bzw. der zweiten Arterie der Person, wobei sowohl das erste Sensorarray als auch das zweite Sensorarray eine Vielzahl von Sensoren (101) umfassen, um jeweils eine Vielzahl von ersten Signalen zu erfassen, die eine Vibration der Haut angeben;
Ableiten (72), sowohl für das erste Sensorarray (31) als auch für das zweite Sensorarray (32), eines zweiten Signals, das die zugehörige Pulswelle darstellt, von der Vielzahl von ersten Signalen; und
Berechnen (73) einer Pulswellenlaufzeit zwischen der ersten Arterie und der zweiten Arterie anhand des für das erste Sensorarray (31) abgeleiteten zweiten Signals und des für das zweite Sensorarray (32) abgeleiteten zweiten Signals;
**dadurch gekennzeichnet, dass**:
jeder der Vielzahl von Sensoren (101) konfiguriert ist, um das erste Signal, das die Vibration der Haut angibt, in mindestens zwei Richtungen zu erfassen;
wobei
jeder der Vielzahl von Sensoren (101) eine Kontaktfläche zum Kontaktieren der Haut der Person umfasst;
die mindestens zwei Richtungen eine X-Achsen-Richtung umfassen, die eine Richtung senkrecht zu der Kontaktfläche ist; und
die Ableitungseinheit (102) konfiguriert ist, um für sowohl das erste Sensorarray (31) als auch das zweite Sensorarray (32) ein erstes Signal aus der Vielzahl von ersten Signalen auszuwählen, und um das zweite Signal von der X-Achsen-Richtungskomponente des ausgewählten ersten Signals abzuleiten.

## Revendications

1. Système (4) pour mesurer une onde pulsée d'un sujet, comprenant :
un premier réseau de capteurs (31) et un second réseau de capteurs (32) pour détecter respectivement une onde pulsée correspondante d'une première et d'une seconde artère du sujet lorsqu'ils sont placés sur la peau du sujet respectivement au-dessus de la première artère et de la seconde artère du sujet, chacun du premier réseau de capteurs et du second réseau de capteurs comprenant une pluralité de capteurs (101) pour acquérir respectivement une pluralité de premiers signaux indiquant une vibration de la peau ;
une unité de dérivation (102) configurée pour dériver, pour chacun du premier réseau de capteurs (31) et du second réseau de capteurs (32), un second signal représentant l'onde pulsée correspondante à partir de la pluralité de premiers signaux ; et
une première unité de calcul (103) configurée pour calculer un temps de transit d'impulsion entre la première artère et la seconde artère à partir du second signal dérivé pour le premier réseau de capteurs (31) et du second signal dérivé pour le second réseau de capteurs (32) ;
**caractérisé en ce que** :
chacun de la pluralité de capteurs (101) est configuré pour acquérir le premier signal indiquant la vibration de la peau dans au moins deux directions ;
dans lequel
chacun de la pluralité de capteurs (101) comprend une surface de contact pour venir en contact avec la peau du sujet ;
les au moins deux directions comprennent une direction d'axe X, qui est une direction perpendiculaire à la surface de contact ; et
l'unité de dérivation (102) est configurée, pour chacun du premier réseau de capteurs (31) et du second réseau de capteurs (32), pour sélectionner un premier signal à partir de la pluralité de premiers signaux, et pour dériver le second signal à partir de la composante de direction d'axe X du premier signal sélectionné.

2. Système (4) selon la revendication 1, dans lequel chacun de la pluralité de capteurs (101) comprend un accéléromètre.

3. Système (4) selon la revendication 2, dans lequel l'accéléromètre est un accéléromètre résistif ou capacitif micro-usiné.

4. Système (4) selon la revendication 1, dans lequel
les au moins deux directions comprennent en outre une direction d'axe Z perpendiculaire à la direction d'axe X ; et
l'unité de dérivation (102) est configurée, pour chacun du premier réseau de capteurs (31) et du second réseau de capteurs (32), pour dériver le second signal en soustrayant la composante de direction d'axe Z du premier signal sélectionné de la composante de direction d'axe X du premier signal sélectionné.

5. Système (4) selon la revendication 1, dans lequel pour chacun du premier réseau de capteurs (31) et du second réseau de capteurs (32), l'unité de dérivation (102) est configurée pour :
déterminer, parmi la pluralité de premiers signaux, un groupe de premiers signaux, dans lequel toutes les corrélations croisées de la composante de direction d'axe X de chacun des premiers signaux dans le groupe avec les composantes de direction d'axe X d'autres premiers signaux dans le groupe sont au-dessus d'un premier seuil prédéterminé, et toutes les corrélations croisées de la composante de direction d'axe X de chacun des premiers signaux dans le groupe avec les composantes de direction d'axe X des premiers signaux en dehors du groupe sont en dessous d'un second seuil prédéterminé, et
sélectionner le premier signal à partir des premiers signaux dans le groupe sur la base d'au moins l'amplitude de signal des premiers signaux.

6. Système (4) selon la revendication 5, dans lequel le rapport amplitude de signal ou signal sur bruit du premier signal sélectionné est le plus élevé parmi les premiers signaux dans le groupe.

7. Système (4) selon la revendication 1, dans lequel l'unité de dérivation (102) est configurée pour dériver, pour chacun de la pluralité de premiers signaux, une composante du premier signal représentant une vibration sensiblement le long de la vibration de l'onde pulsée.

8. Système (4) selon la revendication 1, dans lequel la pluralité de capteurs (101) est fixée sur l'un des deux côtés d'une membrane et l'autre côté de la membrane doit être placé sur la peau du sujet.

9. Système (4) selon la revendication 8, dans lequel la membrane est une membrane mince en métal.

10. Système (4) selon la revendication 1, comprenant en outre :
une unité d'obtention (106) configurée pour obtenir un paramètre (107) indiquant une distance parcourue par l'onde pulsée entre la première artère et la seconde artère ; et
une seconde unité de calcul (104) configurée pour calculer une vitesse d'onde pulsée (105) du sujet, sur la base du temps de transit d'impulsion calculé et du paramètre obtenu.

11. Procédé (7) de mesure d'une onde pulsée d'un sujet, comprenant :
le placement (71) d'un premier réseau de capteurs (31) et d'un second réseau de capteurs (32) respectivement sur la peau du sujet au-dessus d'une première artère et d'une seconde artère du sujet pour détecter respectivement une onde pulsée correspondante de la première et de la seconde artère du sujet, chacun du premier réseau de capteurs et du second réseau de capteurs comprenant une pluralité de capteurs (101) pour acquérir respectivement une pluralité de premiers signaux indiquant une vibration de la peau ;
la dérivation (72), pour chacun du premier réseau de capteurs (31) et du second réseau de capteurs (32), d'un second signal représentant l'onde pulsée correspondante à partir de la pluralité de premiers signaux ; et
le calcul (73) d'un temps de transit d'impulsion entre la première artère et la seconde artère à partir du second signal dérivé pour le premier réseau de capteurs (31) et du second signal dérivé pour le second réseau de capteurs (32) ;
**caractérisé en ce que** :
chacun de la pluralité de capteurs (101) est configuré pour acquérir le premier signal indiquant la vibration de la peau dans au moins deux directions ;
dans lequel
chacun de la pluralité de capteurs (101) comprend une surface de contact pour venir en contact avec la peau du sujet ;
les au moins deux directions comprennent une direction d'axe X, qui est une direction perpendiculaire à la surface de contact ; et
l'unité de dérivation (102) est configurée, pour chacun du premier réseau de capteurs (31) et du second réseau de capteurs (32), pour sélectionner un premier signal à partir de la pluralité de premiers signaux, et pour dériver le second signal de la composante de direction d'axe X du premier signal sélectionné.
